(19) Europäisches Patentamt
European Patent Office
Office européen des brevets

(11) Veröffentlichungsnummer: **0 073 016**
**B1**

(12) # EUROPÄISCHE PATENTSCHRIFT

(45) Veröffentlichungstag der Patentschrift: **27.11.85**

(51) Int. Cl.⁴: **C 07 C 121/80**, A 61 K 31/275

(21) Anmeldenummer: **82107536.3**

(22) Anmeldetag: **18.08.82**

(54) **Neue 1-Aryloxy-3-alkylamino-2-propanole und Verfahren zu ihrer Herstellung.**

(30) Priorität: **26.08.81 DE 3133678**

(43) Veröffentlichungstag der Anmeldung: **02.03.83 Patentblatt 83/9**

(45) Bekanntmachung des Hinweises auf die Patenterteilung: **27.11.85 Patentblatt 85/48**

(84) Benannte Vertragsstaaten: **AT BE CH DE FR GB IT LI LU NL SE**

(56) Entgegenhaltungen:
**EP-A-0 038 936**
**DE-A-2 503 222**

(73) Patentinhaber: **BOEHRINGER INGELHEIM KG, D-6507 Ingelheim am Rhein (DE)**

(72) Erfinder: **Köppe, Herbert, Dr., Neuweg 72, D-6507 Ingelheim (DE)**
Erfinder: **Kummer, Werner, Dr., Georg-Scheuing-Strasse 15, D-6507 Ingelheim (DE)**
Erfinder: **Stähle, Helmut, Dr., Rotweinstrasse 23, D-6507 Ingelheim (DE)**
Erfinder: **Muacevic, Gojko, Dr., In der Dörrwiese 13, D-6507 Ingelheim (DE)**
Erfinder: **Traunecker, Werner, Dr., Birkenweg 1, D-6531 Münster-Sarmsheim (DE)**

**EP 0 073 016 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft neue Verbindungen der Formel

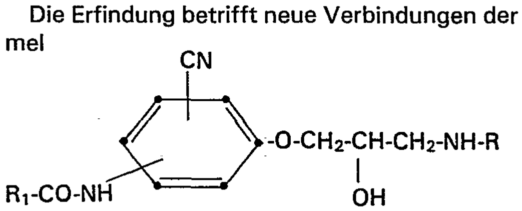

I

in der

$R_1$ einen Cycloalkylrest mit 3 bis 6 C-Atomen, einen Phenylrest, der ggf. durch Methyl, Methoxy, Chlor oder Fluor substituiert sein kann, oder einen Phenoxymethylrest, der ggf. durch Methyl, Chlor oder Brom substituiert sein kann, und

R Isopropyl- oder sec.- oder tert.-Butylrest

bedeutet und deren Säureadditionssalze, die Verwendung dieser Verbindungen in Arzneimitteln sowie ihre Herstellung.

Die neuen Verbindungen können z.B. durch Umsetzung von Verbindungen der Formel II

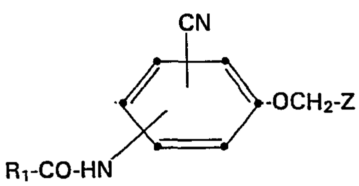

II

in der $R_1$ wie in Formel I definiert ist und Z die Gruppe 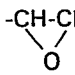 oder $-CHOH-CH_2-Hal$ (Hal = Halogen) bedeutet, mit einem Amin der allgemeinen Formel

$NH_2-R$ III

hergestellt werden.

Die neuen Verbindungen sind als Herz- bzw. Coronartherapeutica oder auch zur Senkung des Blutdrucks verwendbar.

Bevorzugte Bedeutungen für $R_1$ sind substituierte Phenoxymethylreste sowie niedere Cycloalkylreste, insbesondere der Cyclobutylrest.

Die neuen Verbindungen können auf folgende Weise hergestellt werden:

a) Umsetzung einer Verbindung der allgemeinen Formel II

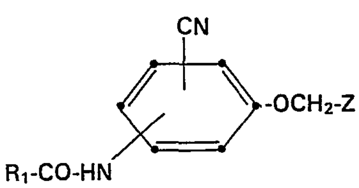

II

in der $R_1$ wie in der Formel I definiert ist und Z die Gruppe 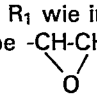 oder $-CHOH-CH_2-Hal$ (Hal = Halogen) bedeutet,
mit einem Amin der allgemeinen Formel III

$NH_2-R$ III

in der R die in Formel I angegebene Bedeutung hat.

b) Hydrolyse eines Oxazolidin-Derivats der allgemeinen Formel IV

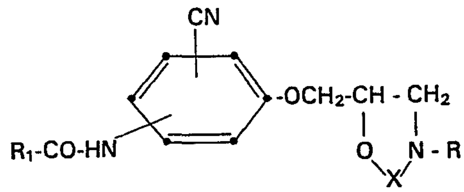

IV

in der $R_1$ und R wie in Formel I definiert sind und X eine -CO-, $-CH_2-$ oder -CH-niederalkyl-Gruppe bedeutet, beispielsweise mit Natron- oder Kalilauge in Wasser oder in einer Alkohol-Wasser-Mischung.

c) Umsetzung einer Verbindung der allgemeinen Formel V

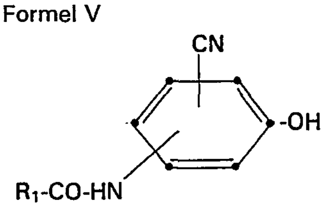

V

in welcher $R_1$ die oben genannte Bedeutung hat, bzw. eines Salzes dieses Phenols, mit einem Azetidinolderivat der allgemeinen Formel VI

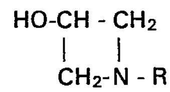

VI

in welcher R die obengenannte Bedeutung hat, in wasserfreiem Medium unter Alkalizusatz.

Die Oxazolidinone der Formel IV (d.h. Verbindungen, bei denen X = -CO- darstellt) sind beispielsweise ausgehend von den Epoxiden der Formel III herstellbar, indem man letztere mit einem (aus Chlorameisensäureäthylester und einem Amin der Formel III darstellbaren) Urethan der Formel VII

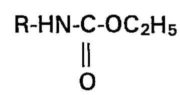

VII

in der R die obenbezeichnete Bedeutung hat, umsetzt.

Die Ausgangsphenole der allgemeinen Formel V und die Azetidinole der allgemeinen Formel VI können nach literaturbekannten Methoden [letztere s. beispielsweise Chem. pharm. Bull. (Japan), Vol. 22 (7), 1974, Seite 1490] hergestellt werden.

Die erfindungsgemässen Verbindungen besitzen ein asymmetrisches C-Atom an der CHOH-Gruppe und kommen daher als Racemat wie auch in Form der optischen Antipoden vor. Letztere können ausser durch Racematentrennung mit üblichen Hilfssäuren wie Dibenzoyl- (bzw. Di-p-Toluyl-) D-Weinsäure oder D-3-Bromcampher-8-sulfonsäure auch durch Einsetzen von optisch aktivem Ausgangsmaterial erhalten werden.

Die erfindungsgemässen 1-Aryloxy-3-alkylamino--2-propanole der allgemeinen Formel I können in

üblicher Weise in ihre physiologisch verträglichen Säureadditionssalze überführt werden. Geeignete Säuren sind beispielsweise Salzsäure, Bromwasserstoffsäure, Schwefelsäure, Methansulfonsäure, Maleinsäure, Essigsäure, Oxalsäure, Milchsäure, Weinsäure oder 8-Chlortheophyllin.

Die Verbindungen der allgemeinen Formel I bzw. deren physiologisch verträgliche Säureadditionssalze haben im Tierversuch wertvolle therapeutische, insbesondere β-adrenolytische Eigenschaften gezeigt und können daher beispielsweise zur Behandlung oder Prophylaxe koronarer Herzkrankheiten, insbesondere der Angina pectoris und zur Behandlung von Herzarrhythmien, insbesondere von Tachycardien, in der Humanmedizin eingesetzt werden.

Therapeutisch besonders interessant sind die blutdrucksenkenden Eigenschaften und calciumantagonistischen Effekte der Verbindungen der Formel I. Zudem entfalten sie günstige metabolische Eigenschaften. Die Verbindungen haben gegenüber bekannten β-Rezeptorenblockern, z.B. dem ähnlich strukturierten Handelsprodukt 1-(2-Acetyl-4-butyroylaminophenoxy)-3-isopropylamino-2-propanol (Acebutolol) den Vorteil beträchtlich verminderter Toxizität, besserer Wirkung und hervorragender Organselektivität.

Gegenüber der ähnlich strukturierten Verbindung 1-(2-Cyano-4-propionamido-phenoxy)-3-tert.-butylamino-2-propanol (USA-Patentschrift 3712927) haben die erfindungsgemässen Verbindungen den Vorteil hervorragender Organselektivität.

Die Messung dieser Parameter erfolgte dabei nach den folgenden Vorschriften:

1. *Hemmung der Isoprenalintachycardie* (aludrinantagonistische Wirkung)

*Methode*: Hemmung der tachycarden Reaktion auf eine Standard-Dosis Isoprenalin und Einfluss auf die basale Herzfrequenz durch steigende i.V. Dosen eines β-Adrenolytikums.

*Tiermaterial*: Meerschweinchen beiderlei Geschlechts mit Körpergewichten von 270 - 350 g, Gruppenhaltung, Standard-Kost und Wasser bis zum Versuchsbeginn ad libitum. 16 Stunden vor dem Versuchsanfang Futterentzug.

*Narkose*: Äthylurethan 1,75 g/kg als 20%ige Lösung intraperitoneal, gegebenenfalls wurde nachinjiziert.

*Präparation*: Kanülierung einer Vena jugularis exterior für intravenöse Injektionen: Einbinden einer Trachealkanüle und künstliche Beatmung; subcutane Nadelelektroden zur Aufnahme des EKG, in der Regel Extremitätenableitung II, Registriergeschwindigkeit 25 mm/sec; Rektalthermometer zur Kontrolle der Körpertemperatur, die mit einer Wärmelampe (Infrarotstrahler) auf 34 bis 36°C mit Hilfe einer elektronischen Automatikeinrichtung konstant gehalten wird.

*Versuchsablauf:* Die Herzfrequenz wird durch Auszählen der R-Zacken im EKG bestimmt, jeweils aus einer 3 bis 4 sec langen Registrierzeit, etwa 30 min nach der Präparationen wird im Abstand von 2 min 5mal die normale Herzfrequenz gemessen und gemittelt. Anschliessend wird als adrenerges Stimulans 1 µg/kg Isoprenalin i.v. injiziert und danach 3 min lang alle 30 sec die Herzfrequenz erneut registriert. Die Isoprenalininjektionen werden während des ganzen Versuches im Abstand von 30 min wiederholt. Bleibt die Spontanfrequenz etwa konstant und ist die tachycarde Reaktion auf die ersten 2 bis 3 Isoprenalin-Gaben gleichmässig, dann wird 15 min nach der letzten und 15 min vor der nächsten Isoprenalin-Reaktion die erste Dosis der Prüfsubstanz i.v. injiziert. Weitere in geometrischer Reihe ansteigende Dosen der Prüfsubstanz folgen in Abständen von 60 min bis eine markante Hemmung der Isoprenalin-Tachycardie erreicht ist.

2. *Prüfung auf Cardioselektivität am wachen Meerschweinchen*

*Prinzip*: Nach der Methode von D. Dunlop und R.G. Shanks [brit. J. Pharmacol. 32, 201 (1968)] werden wache Meerschweinchen einer tödlichen Dosis eines Histaminaerosols ausgesetzt. Durch Vorbehandlung mit Isoprenalin werden die Tiere vor der letalen Wirkung des Histamins geschützt. Ein β-Adrenolytikum hebt die Isoprenalinwirkung auf, so dass der Schutz vor dem Histaminbronchospamus verloren geht, falls es sich um eine nicht cardio selektive Substanz handelt. Zeigt eine am Herzen wirksame β-adrenolytische Substanz in diesem Test keinen Antagonimus gegen Isoprenalin, dann kann Cardioselektivität (für sog. $\beta_1$-Rezeptoren) angenommen werden.

*Tiermaterial*: Meerschweinchen beiderlei Geschlechts (6 Tiere pro Dosis), mit 350 bis 400 g Körpergewicht, Gruppenhaltung, Standard Futter und Wasser bis zum Versuchsbeginn ad libitum. 16 Stunden vor dem Versuchsanfang Futterentzug.

*Versuchsverlauf*: Gruppen von je 6 Tieren (3 männliche + 3 weibliche) werden s.c. mit 5 oder mehr verschiedenen Dosen des β-Adrenolytikums behandelt. Fünfzehn Minuten später erhalten sie 0,1 mg/kg Isoprenalin contralateral s.c. injiziert. Nach weiteren 15 Minuten werden die Tiere in die zylindrische Kammer von 2 Liter Inhalt gesetzt, 45 Sekunden lang einem wässrigen Histaminaerosol (1,25%-ig) ausgesetzt und anschliessend wird die Mortalität ausgewertet.

*Auswertung*: Die Letalität wird gegen den Logarithmus der Dosis aufgetragen und die $LD_{50}$ nach J. LITCHFIELD und F. WILCOXON (J. Pharmacol. Exp. Therap. *96*, 99-113, 1949) ermittelt. Mit der $LD_{50}$ aus diesem Versuch und der cardialen $ED_{50}$ aus dem Versuch der Hemmung der Isoprenalintachycardie (narkot. Meerschweinchen) wird ein Selektivitätsquotient $\left(\frac{\text{LD 50}}{\text{ED 50}}\right)$ gebildet. Eine Substanz wird als cardioselektiv angesehen, wenn der Quotient grösser als 1 ist.

Als wertvoll haben sich dabei insbesondere solche Verbindungen der allgemeinen Formel I herausgestellt, bei denen R einen Isopropyl- oder einen tertiär-Butylaminorest bedeutet (substituierte p-Acylamino-1-Phenoxy-3-isopropyl- bzw. tert.-butyl-amino-2-propanole). Besonders wertvoll sind insbesondere das 1-[2-Cyano-4-(3'-methylphenoxyacetylami-

no)-phenoxy]-3-tertiärbutyl-amino-2-propanol und das 1-(2-Cyano-4-cyclobutancarbonylaminophenoxy)-3-tertiärbutylamino-2-propanol und deren Salze.

Die Einzeldosis der erfindungsgemässen Substanzen liegt bei 1 bis 300 mg, vorzugsweise 5 bis 100 mg (oral) bzw. 1 bis 20 mg (parenteral).

Die erfindungsgemässen Wirkstoffe können in die üblichen galenischen Anwendungsformen, wie Tabletten, Dragées, Lösungen, Emulsionen, Pulver, Kapseln oder Depotformen gebracht werden, wobei zu deren Herstellung die üblichen pharmazeutischen Hilfsstoffe sowie die üblichen Fertigungsmethoden herangezogen werden können. Entsprechende Tabletten können beispielsweise durch Mischen der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung eines Depoteffekts, wie Carboxypolymethylen, Carboxymethylcellulose, Celluloseacetatphthalat, oder Polyvinylacetat erhalten werden.

Die Tabletten können auch aus mehreren Schichten bestehen. Entsprechend können Dragées durch Überziehen von analog den Tabletten hergestellten Kernen mit üblicherweise in Dragéeüberzügen verwendeten Mitteln, beispielsweise Kollidon oder Schellack, Gummi arabicum, Talk, Titandioxid oder Zucker, hergestellt werden. Zur Erzielung eines Depoteffekts oder zur Vermeidung von Inkompatibilitäten kann der Kern auch aus mehreren Schichten bestehen. Desgleichen kann auch die Dragéehülle zur Erzielung eines Depoteffekts aus mehreren Schichten bestehen, wobei die oben bei den Tabletten erwähnten Hilfsstoffe verwendet werden können.

Säfte der erfindungsgemässen Wirkstoffe bzw. Wirkstoffkombinationen können zusätzlich noch ein Süssungsmittel, wie Saccharin, Cyclamat, Glycerin oder Zucker, sowie ein geschmackverbesserndes Mittel, z.B. Aromastoffe, wie Vanillin oder Orangenextrakt, enthalten.

Sie können ausserdem Suspendierhilfsstoffe oder Dickungsmittel, wie Natriumcarboxymethyl-cellulose, Netzmittel, beispielsweise Kondensationsprodukte von Fettalkoholen mit Äthylenoxid, oder Schutzstoffe, wie p-Hydroxybenzoate, enthalten.

Injektionslösungen werden in üblicher Weise, z.B. Unter Zusatz von Konservierungsmitteln, wie p-Hydroxybenzoaten, oder Stabilisatoren, wie Komplexonen, hergestellt und in Injektionsflaschen oder Ampullen abgefüllt.

Die Wirkstoffe bzw. Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen der dafür vorgesehenen Wirkstoffe bzw. Wirkstoffkombinationen mit üblichen Trägermitteln, wie Neutralfetten oder Polyäthylenglykol bzw. dessen Derivaten, herstellen.

Die erfindungsgemässen Verbindungen sind auch für die Kombination mit anderen pharmakodynamisch wirksamen Stoffen wie z.B. Coronardilatatoren, Sympaticomimetica, Herzglykosiden oder Tranquilizern geeignet.

Die folgenden Beispiele erläutern die Erfindung, ohne sie zu beschränken:

*Beispiel 1*

*1-[2-Cyano-4-(3'-methylphenoxyacetylamino)--phenoxy]-3-tert.-butylamino-2-propanol*

7 g (0,021 Mol) 1-[2-Cyano-4-(3'-methylphenoxyacetylamino)-phenoxy]-2,3-epoxypropan werden in 80 ml Äthanol gelöst und nach Zugabe von 2,2 ml (0,021 Mol) tert.-Butylamin 90 Minuten unter Rückfluss zum Sieden erhitzt. Nach Abdestillieren des Lösungsmittels wird der verbleibende Rückstand mit Essigester digeriert und der feste Anteil abgesaugt. Die feinkristalline Base wird zweimal aus Essigester unter Zugabe von Hexan umkristallisiert. Ausbeute 2,6 g, Fp.: 130-131 °C.

*Beispiel 2*

*1-(2-Cyano-4-cyclobutancarbonylamino-phenoxy)--3-tert.-butylamino-2-propanol*

7,5 g (0,024 Mol) 1-(2-Cyano-4-cyclobutancarbonylaminophenoxy)-3-chloro-2-propanol werden nach Lösen in 80 ml Äthanol mit 8,7 g (0,12 Mol) tert.-Butylamin vermischt und vier Stunden unter Rückfluss zum Sieden erhitzt. Das Lösungsmittel wird i.V. abdestilliert, der verbleibende Rückstand mit verdünnter Salzsäure unter Zugabe von Äther geschüttelt und die organische Phase abgetrennt. Die wässrige Phase wird mit Äther gewaschen und mit $NH_4OH$ alkalisch gestellt. Die ausfallende Base wird mit Essigester extrahiert und die organische Phase über $MgSO_4$ getrocknet. Der Essigester wird abdestilliert und der kristalline Rückstand zweimal aus Acetonitril umkristallisiert. Ausbeute: 5,7 g, Fp.: 110-113°C.

*Beispiel 3*

*1-[2-Cyano-4-(4'-chlorbenzoylamino)-phenoxy]-3--tert.-butylamino-2-propanol*

12 g 1-[2-Cyano-4-(4'-chlorbenzoyl-amino)-phenoxy]-3-chlor-2-propanol werden in 100 ml Äthanol gelöst, 15 ml tert.-Butylamin zugegeben und drei Stunden unter Rückfluss zum Sieden erhitzt. Nach Abdestillieren des Äthanols wird der Rückstand mit verdünnter Salzsäure angesäuert, die wässrige Lösung mit Äther ausgeschüttelt und die wässrige Phase mit NaOH alkalisch gestellt. Die Base wird in Essigester aufgenommen, die organische Phase mit Wasser gewaschen, über $Na_2SO_4$ getrocknet und das Lösungsmittel abdestilliert. Der Rückstand wird über eine Kieselgelsäule gereinigt. Die feste Base wird aus Isopropanol umkristallisiert. Ausbeute 4 g, Fp.: 182-183°C.

*Beispiel 4*

*1-[2-Cyano-4-(4'-methylbenzoylamino)-phenoxy]--3-isopropylamino-2-propanol-hydrochlorid*

3 g (0,009 Mol) 1-[2-Cyano-4-(4'-methylbenzoyl-

amino)-phenoxy]-3-chlor-2-propanol werden in 80 ml Äthanol gelöst, 3,8 ml (0,045 Mol) Isopropylamin zugegeben und zwei Stunden unter Rückfluss zum Sieden erhitzt. Nach Abdestillieren des Methanols wird der Rückstand in Salzsäure gelöst, mit Essigester extrahiert und anschliessend die wässrige Phase mit NaOH alkalisch gestellt. Die freie Base wird mit Essigester mehrfach extrahiert und nach Waschen mit Wasser über $Na_2SO_4$ getrocknet. Durch Abdestillieren des Essigesters erhält man einen Rückstand, der mit Acetonitril digeriert, dann abgesaugt und in Acetonitril aufgeschlämmt wird. Nach Zugabe von alkoholischer HCl tritt Lösung ein. Die Zugabe von Äther leitet die Kristallisation des Hydrochlorids ein, die durch Abkühlung beschleunigt wird. Das Hydrochlorid wird durch Lösen in Äthanol und Zugabe von Äther umkristallisiert. Ausbeute: 2 g, Fp.: 193 bis 195°C.

*Beispiel 5*

*1-[2-Cyano-4-(4'-methoxybenzoylamino)-phenoxy]-3-tert.-butylamino-2-propanol*

14 g 1-[2-Cyano-4-(4'-methoxybenzoylamino)--phenoxy]-3-chlor-2-propanol werden nach Lösen in 100 ml Äthanol und Zugabe von 20 ml tert.-Butylamin vier Stunden unter Rückfluss zum Sieden erhitzt. Nach Abdestillieren des Äthanols wird der verbleibende Rückstand in verdünnter Salzsäure gelöst, mit Äther ausgeschüttelt, die wässrige Phase mit NaOH alkalisch gestellt und die freie Base in Essigester aufgenommen. Die organische Phase wird mit Wasser gewaschen, über $Na_2SO_4$ getrocknet, Essigester abdestilliert und der verbleibende Rückstand über eine Kieselgelsäule (Essigester/Isopropanol/$NH_4OH$ 70/30/5) gereinigt. Die sodann isolierte Base wird aus Essigester und Zugabe von Petroläther (Kp 40°C) umkristallisiert. Ausbeute: 3,2 g, Fp.: 108-110°C.

Entsprechend der Vorschrift des Beispiels 2 wurden aus dem entsprechenden p-Acylamino-phenoxy-2-hydroxy-3-chlorpropan der Formel II sowie dem entsprechenden Alkylamin der Formel II in Äthanol am Rückfluss die folgenden Substanzen der Formel I synthetisiert: (CN in 2-Stellung, Acylaminorest in 4-Stellung der Phenoxygruppe)

| Beispiel Nr. | $R_1$ | R | Fp. °C (wenn nicht anders angegeben Hydrochlorid) |
|---|---|---|---|
| 6 | 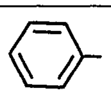 | tert.$C_4H_9$ | 165-166 |
| 7 | 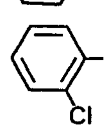 | tert.$C_4H_9$ | 117-120 |
| 8 | 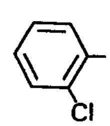 | iso $C_3H_7$ | 159-162 (Base) |
| 9 | 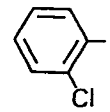 | sec.$C_4H_9$ | 127-130 (Base) |
| 10 | 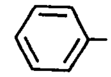 | iso $C_3H_7$ | 214-216 |
| 11 | 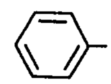 | tert.$C_4H_9$ | 151-152 (Base) |
| 12 | 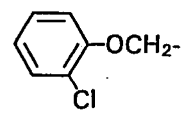 | tert.$C_4H_9$ | 124-125 (Base) |
| 13 | 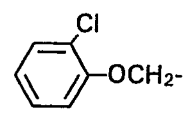 | iso $C_3H_7$ | 130-131 (Base) |
| 14 | 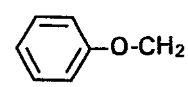 | sec.$C_4H_9$ | 139-140 (Base) |
| 15 | 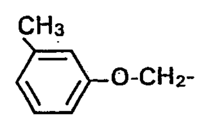 | iso $C_3H_7$ | 172-173 |
| 16 | 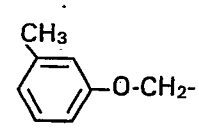 | sec.$C_4H_9$ | 134-135 (Base) |
| 17 | 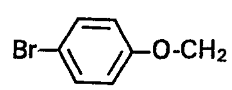 | iso $C_3H_7$ | 240-242 |
| 18 | 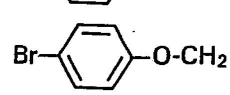 | sec.$C_4H_9$ | 216-218 |
| 19 | 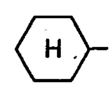 | iso $C_3H_7$ | 142-143 (Base) |
| 20 | 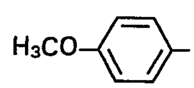 | iso $C_3H_7$ | 157-159 (Base) |
| 21 | 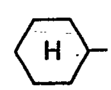 | tert.$C_4H_9$ | 143-144 |
| 22 | 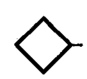 | iso $C_3H_7$ | 120-123 (Base) |
| 23 | 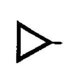 | iso $C_3H_7$ | 158-159 (Base) |
| 24 | 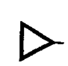 | tert.$C_4H_9$ | 121-124 (Base) |
| 25 | 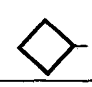 | sec.$C_4H_9$ | 83- 86 (Base) |

| Beispiel Nr. | $R_1$ | R | Fp. °C (wenn nicht anders angegeben Hydrochlorid) |
|---|---|---|---|
| 26 | 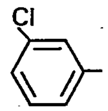 | tert.$C_4H_9$ | 182-184 |
| 27 | 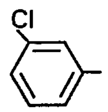 | iso $C_3H_7$ | 194-196 |
| 28 | 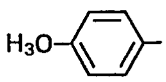 | tert.$C_4H_9$ | 155-158 |
| 29 | 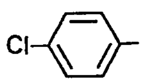 | iso $C_3H_7$ | 164-165 (Base) |
| 30 | 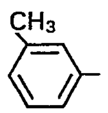 | iso $C_7H_7$ | 154-155 (Base) |
| 31 | 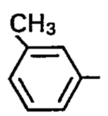 | tert.$C_4H_9$ | 126-127 (Base) |
| 32 | 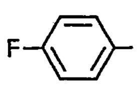 | tert.$C_4H_9$ | 191-193 (Base) |
| 33 | 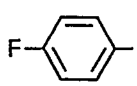 | iso $C_3H_7$ | 203-205 |

1 B. *Formulierungsbeispiele*

1. *Tabletten*

| | |
|---|---|
| 1-[2-Cyano-4-(3'-methylphenoxyacetylamino)-phenoxy-3-tert.-butylamino-2-propanol | 40,0 mg |
| Maisstärke | 164,0 mg |
| sek. Calciumphosphat | 240,0 mg |
| Magnesiumstearat | 1,0 mg |
| | 445,0 mg |

Herstellung:
Die einzelnen Bestandteile werden intensiv miteinander vermischt und die Mischung in üblicher Weise granuliert. Das Granulat wird zu Tabletten von 445 mg Gewicht verpresst, von denen jede 40 mg Wirkstoff enthält.

Anstelle des in diesem Beispiel genannten Wirkstoffs können auch die Substanzen 1-[2-Cyano-4-(4'-chlorbenzoylamino)-phenoxy]-3-(tert.-butylamino)-2-propanol und 1-[2-Cyano-4-(4'-methylbenzoylamino)-phenoxy]-3-(isopropylamino)-2-propanol-hydrochlorid in gleicher Menge verwendet werden.

2. *Gelatine-Kapseln*

Der Inhalt der Kapsel setzt sich wie folgt zusammen:

| | |
|---|---|
| 1-(2-Cyano-4-cyclobutancarbonylamino-phenoxy)-3-(tert.-butylamino)-2-propanol · HCl | 25,0 mg |
| Maisstärke | 175,0 mg |
| | 200,0 mg |

Herstellung:
Die Bestandteile des Kapselinhalts werden intensiv vermischt und 200 mg-Portionen der Mischung werden in Gelatine-Kapseln geeigneter Grösse abgefüllt. Jede Kapsel enthält 25 mg des Wirkstoffs.

3. *Injektionslösung*

Die Lösung wird aus folgenden Bestandteilen hergestellt:

| | | |
|---|---|---|
| 1-[2-Cyno-4-(4'-methoxybenzoylamino)-phenoxy]-3-tert.-butylamino-2-propanol | | 2,5 Teile |
| Natriumsalz der EDTA (Äthylendiamintetraessigsäure) | | 0,2 Teile |
| dest. Wasser | ad | 100,0 Teile |

Herstellung:
Der Wirkstoff und das EDTA-Salz werden in genügend Wasser gelöst und mit Wasser auf das gewünschte Volumen aufgefüllt. Die Lösung wird frei von suspendierten Partikeln filtriert und in 1 ccm-Ampullen unter aseptischen Bedingungen abgefüllt. Zuletzt werden die Ampullen sterilisiert und verschlossen. Jede Ampulle enthält 25 mg Wirkstoff.

4. *Depotdragées*

Kern:

| | |
|---|---|
| (—)-1-[2-Cyano-4-(3'-methylphenoxy-acetylamino)-phenoxy]-3-tert.-butylamino-2-propanol | 25,0 g |
| Carboxymethylcellulose (CMC) | 295,0 g |
| Stearinsäure | 20,0 g |
| Celluloseacetatphthalat (CAP) | 40,0 g |
| | 380,0 g |

Herstellung:
Der Wirkstoff, die CMC und die Stearinsäure werden intensiv gemischt und die Mischung in üblicher Weise granuliert, wobei man eine Lösung des CAP in 200 ml eines Gemisches aus Äthanol/Äthylacetat verwendet. Das Granulat wird dann zu 380 mg-Kernen verpresst, die in üblicher Weise mit einer zuckerhaltigen 5%igen Lösung von Polyvinylpyrrolidon in Wasser überzogen werden. Jedes Dragée enthält 25 mg Wirkstoff.

5. *Tabletten*

| | |
|---|---|
| 1-(2-Cyano-4-cyclobutancarbonyl-aminophenoxy)-3-tert.-butylamino-2-propanol | 35,0 g |
| 2,6-Bis-(diäthanolamino)-4,8-dipiperidinopyrimido-[5,4-d]-pyrimidin | 75,0 g |

| | |
|---|---|
| Milchzucker | 164,0 g |
| Maisstärke | 194,0 g |
| kolloidale Kieselsäure | 14,0 g |
| Polyvinylpyrrolidon | 6,0 g |
| Magnesiumstearat | 2,0 g |
| lösliche Stärke | 10,0 g |
| | 500,0 g |

Anstelle des in diesem Beispiel genannten β-adrenolytisch wirksamen Stoffs kann auch die Substanz 1-[2-Cyano-4-(3'-methylphenoxyacetylamino)-phenoxy]-3-(tert.-butylamino)-2-propanol in gleicher Menge verwendet werden.

Herstellung:
Der Wirkstoff wird zusammen mit dem Milchzucker, der Maisstärke, der kolloidalen Kieselsäure und dem Polyvinylpyrrolidon nach intensiver Durchmischung in üblicher Weise granuliert, wobei man eine wässrige Lösung der löslichen Stärke verwendet. Das Granulat wird mit dem Magnesiumstearat gemischt und zu 1000 Tabletten von je 500 mg Gewicht verpresst, die je 35 mg des ersten und 75 mg des zweiten Wirkstoffs enthalten.

**Patentansprüche für die Vertragsstaaten:**
BE, CH, DE, FR, GB, IT, LI, LU, NL, SE

1. Phenoxypropanolamine der allgemeinen Formel (I)

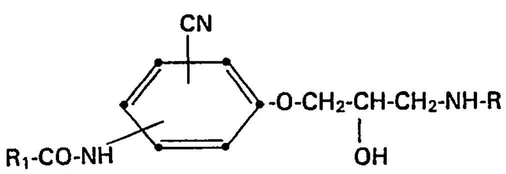
I

worin
$R_1$ einen Cycloalkylrest mit 3 bis 6 C-Atomen oder einen Phenylrest, der ggf. durch Methyl, Methoxy, Chlor oder Fluor substituiert sein kann, oder einen Phenoxymethylrest, der ggf. durch Methyl, Chlor oder Brom substituiert sein kann, und
R einen Isopropyl- oder sec.- oder tert.-Butylrest bedeutet und deren Säureadditionssalze.

2. 1-[2-Cyano-4-(3'-methylphenoxyacetylamino)-phenoxy]-3-tert.-butylamino-2-propanol bzw. seine Salze.

3. 1-(2-Cyano-4-cyclobutancarbonylamino-phenoxy)-3-tert.-butylamino-2-propanol bzw. seine Salze.

4. Pharmazeutische Zubereitungen enthaltend Substanzen der allgemeinen Formel (I) nach Anspruch 1 bzw. ihre Säureadditionssalze in Kombination mit üblichen Hilfs- und/oder Trägerstoffen.

5. Pharmazeutische Präparate, enthaltend Substanzen der allgemeinen Formel (I) nach Anspruch 1 bzw. ihre Säureadditionssalze in Kombination mit anderen pharmazeutischen Wirkstoffen sowie üblichen Hilfs- und/oder Trägerstoffen.

6. Verbindungen nach Anspruch 1 zur Verwendung als Wirkstoffe für Arzneimittel zur Behandlung und Prophylaxe von Erkrankungen der Herzkranzgefässe.

7. Verfahren zur Herstellung von pharmazeutischen Zubereitungen nach Anspruch 4 oder 5, dadurch gekennzeichnet, dass man Substanzen der allgemeinen Formel (I) nach Anspruch 1 bzw. ihre Säureadditionssalze sowie üblichen galenischen Hilfs- und/oder Trägerstoffen zu üblichen pharmazeutischen Anwendungsformen verarbeitet.

8. Verfahren zur Herstellung von Verbindungen der allgemeinen Formel (I) nach Anspruch 1, dadurch gekennzeichnet, dass man

a) eine Verbindung der allgemeinen Formel (II)

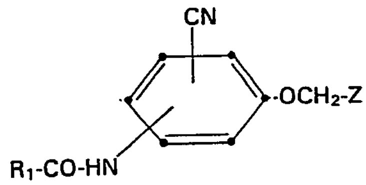
II

in der $R_1$ wie in der Formel (I) definiert ist und Z die Gruppe 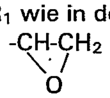 oder $-CHOH-CH_2-Hal$ (Hal = Halogen) bedeutet,
mit einem Amin der allgemeinen Formel (III)

$NH_2-R$ III

in der R wie in Formel (I) definiert ist, umsetzt, oder dass man

b) ein Oxazolidin-Derivat der allgemeinen Formel (IV)

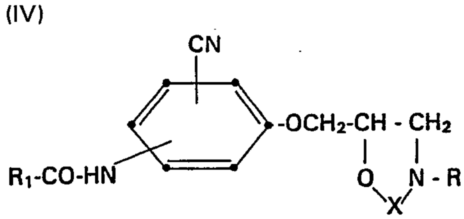
IV

in der $R_1$ und R wie in Formel I und X ein -CO-, $-CH_2$- oder -CH-Niederalkylgruppe bedeutet, definiert sind, hydrolysiert, oder dass man

c) eine Verbindung der allgemeinen Formel (V)

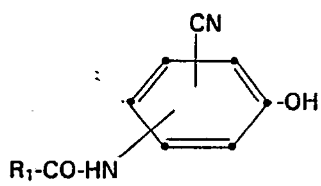
V

in welcher $R_1$ wie in Formel I definiert ist (oder ein Salz dieser Verbindung) mit einem Azetidinolderivat der allgemeinen Formel (VI)

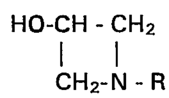
VI

in welcher R wie in Formel (I) definiert ist, umsetzt, und die nach einem Verfahren a) bis c) erhaltenen Verbindungen gewünschtenfalls in ihre Säureadditionssalze überführt.

**Patentansprüche für den Vertragsstaat:** AT

1. Verfahren zur Herstellung von Phenoxypropanolamine der allgemeinen Formel (I)

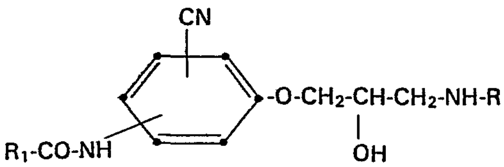

I

worin

$R_1$ einen Cycloalkylrest mit 3 bis 6 C-Atomen oder einen Phenylrest, der ggf. durch Methyl, Methoxy, Chlor oder Fluor substituiert sein kann, oder einen Phenoxymethylrest, der ggf. durch Methyl, Chlor oder Brom substituiert sein kann, und

R einen Isopropyl- oder sec.- oder tert.-Butylrest

bedeutet und deren Säureadditionssalze, dadurch gekennzeichnet, dass man

a) eine Verbindung der allgemeinen Formel (II)

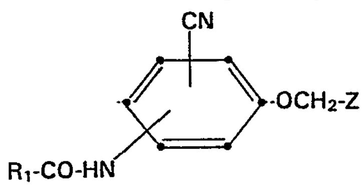

II

in der $R_1$ wie in Formel (I) definiert ist und Z die Gruppe 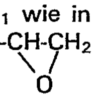 oder $-CHOH-CH_2-Hal$ (Hal = Halogen) bedeutet,

mit einem Amin der allgemeinen Formel (III)

$NH_2-R$ III

in der R wie in Formel (I) definiert ist, umsetzt, oder dass man

b) ein Oxazolidin-Derivat der allgemeinen Formel (IV)

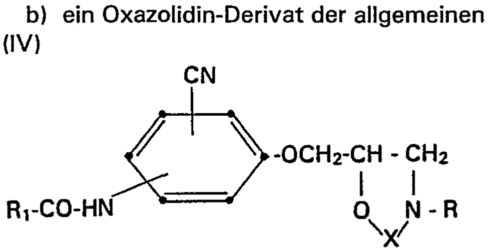

IV

in der $R_1$ und R wie in Formel I und X ein -CO-, $-CH_2-$ oder -CH-Niederalkylgruppe bedeutet, definiert sind, hydrolysiert, oder dass man

c) eine Verbindung der allgemeinen Formel (V)

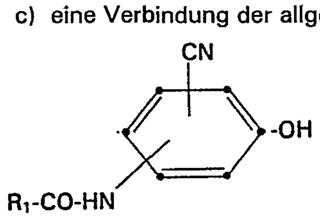

V

in welcher $R_1$ wie in Formel I definiert ist (oder ein Salz dieser Verbindung) mit einem Azetidinolderivat der allgemeinen Formel (VI)

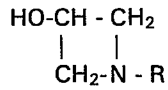

VI

in welcher R wie in Formel (I) definiert ist, umsetzt, und die nach einem Verfahren a) bis c) erhaltenen Verbindungen gewünschtenfalls in ihre Säureadditionssalze überführt.

2. Verfahren nach Anspruch 1, dadurch gekennzeichnet, dass 1-[2-Cyano-4-(3'-methylphenoxyacetylamino)-phenoxy]-3-tert.-butylamino-2-propanol bzw. seine Salze hergestellt wird.

3. Verfahren nach Anspruch 1, dadurch gekennzeichnet, dass 1-(2-Cyano-4-cyclobutancarbonylamino-phenoxy)-3-tert.-butylamino-2-propanol bzw. seine Salze hergestellt wird.

4. Verfahren zur Herstellung von pharmazeutischen Zubereitungen, dadurch gekennzeichnet, dass man Substanzen der allgemeinen Formel (I) nach Anspruch 1 bzw. ihre Säureadditionssalze sowie üblichen galenischen Hilfs- und/oder Trägerstoffen zu üblichen pharmazeutischen Anwendungsformen verarbeitet.

5. Verfahren zur Herstellung von pharmazeutischen Zubereitungen, dadurch gekennzeichnet, dass man Substanzen der allgemeinen Formel (I) nach Anspruch 1 bzw. ihre Säureadditionssalze mit anderen pharmazeutischen Wirkstoffen sowie üblichen galenischen Hilfs- und/oder Trägerstoffen zu üblichen pharmazeutischen Anwendungsformen verarbeitet.

**Claims for the Contracting states:**
BE, CH, DE, FR, GB, IT, LI, LU, NL, SE

1. Phenoxypropanolamines of general formula (I)

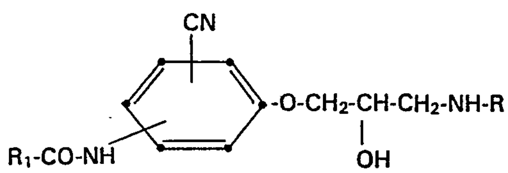

I

wherein

$R_1$ represents a cycloalkyl group containing 3 to 6 carbon atoms or a phenyl group which may optionally be substituted by methyl, methoxy, chlorine or fluorine, or a phenoxymethyl group which may optionally be substituted by methyl, chlorine or bromine, and

R represents an isopropyl or sec.- or tert.-butyl group, and the acid addition salts thereof.

2. 1-[2-Cyano-4-(3'-methylphenoxyacetylamino)-phenoxy]-3-tert.-butylamino-2-propanol or the salts thereof.

3. 1-(2-Cyano-4-cyclobutane-carbonylamino-phenoxy)-3-tert.-butylamino-2-propanol and the salts thereof.

4. Pharmaceutical preparations containing substances of general formula I as claimed in claim 1 or their acid addition salts in conjunction with conventional excipients and/or carriers.

5. Pharmaceutical preparations containing substances of general formula I as claimed in claim 1

or their acid addition salts in conjunction with other pharmaceutical active substances and conventional excipients and/or carriers.

6. Compounds as claimed in claim 1 for use as active substances for pharmaceutical compositions for the treatment and prophylaxis of diseases of the coronary blood vessels.

7. Process for preparing pharmaceutical preparations as claimed in claim 4 or 5, characterised in that substances of general formula I as claimed in claim 1 or their acid addition salts and conventional galenic excipients and/or carriers are processed to form conventional pharmaceutical forms for administration.

8. Process for preparing compounds of general formula I as claimed in claim 1, characterised in that

a) a compound of general formula II

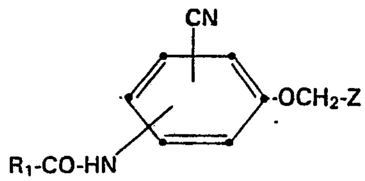

II

wherein $R_1$ is defined as in formula I and Z represents the group 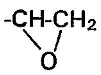 or $-CHOH-CH_2Hal$ (Hal = halogen) is reacted with an amine of general formula III

$NH_2-R$ III

wherein R is defined as in formula I, or

b) an oxazolidine derivative of general formula IV

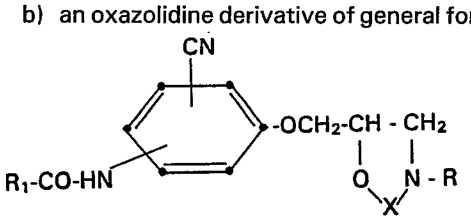

IV

wherein $R_1$ and R are defined as in formula I and X represents a -CO-, $-CH_2-$ or -CH- lower alkyl group, is hydrolysed, or

c) a compound of general formula V

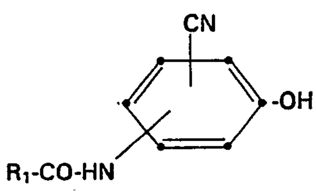

V

wherein $R_1$ is defined as in formula I, or a salt of this compound, is reacted with an azetidinol derivative of general formula VI

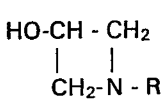

VI

wherein R is defined as in formula I, and, if desired, the compounds obtained according to one of the processes a) to c) are converted into the acid addition salts thereof.

**Claims for the Contracting state:** AT

1. Process fo the preparation of phenoxypropanolamines of general formula I

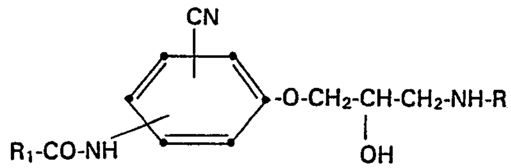

I

wherein

$R_1$ is a cycloalkylgroup containing 3 to 6 carbon atoms or a phenyl group which may optionally be substituted by methyl, methoxy, chlorine or fluorine, or a phenoxymethylgroup which may optionally be substituted by methyl, chlorine or bromine, and

R represents an isopropyl or sec.- or tert.-butyl group, and the acid addition salts thereof,

characterised in that

a) a compound of general formula II

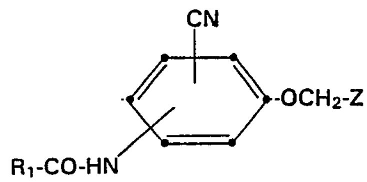

II

wherein $R_1$ is defined as in formula I and Z represents the group 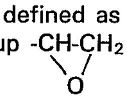 or $-CHOH-CH_2Hal$ (Hal = halogen) is reacted with an amine of general formula III

$NH_2-R$ III

wherein R is defined as in formula I, or

b) an oxazolidine derivative of general formula IV

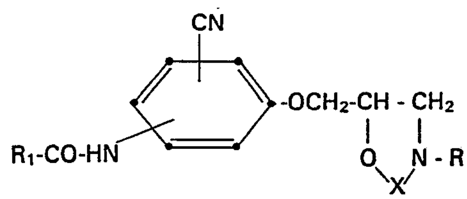

IV

wherein $R_1$ and R are defined as in formula I and X represents -CO-, $-CH_2-$ or -CH- loweralkyl group, is hydrolysed, or

c) a compound of general formula V

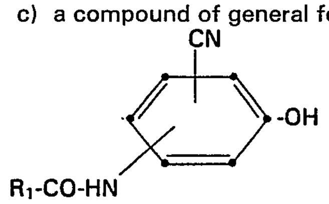

V

wherein $R_1$ is defined as in formula I, or a salt of this compound, is reacted with an azetidinol derivative of general formula VI

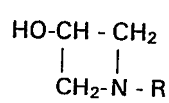

VI

wherein R is defined as in formula I,
and, if desired, the compounds obtained according to one of the processes a) to c) are converted into the acid addition salts thereof.

2. Process according to claim 1, characterised in that the compound 1-[2-Cyano-4-(3'-methylphenoxiacetylamino)-phenoxy]-3-tert.-butylamino-2--propanol or the salts thereof is produced.

3. Process according to claim 1, characterised in that the compound 1-(2-Cyano-4-cyclobutancarbonylamino-phenoxy)-3-tert.-butylamino-2-propanol or the salts thereof is produced.

4. Process for the preparation of a pharmaceutical composition characterised in that substances of general formula I according to claim 1 or the acid addition salts thereof, and conventional galenic excipients and/or carriers are processed to form conventional pharmaceutical forms for administration.

5. Process for the preparation of a pharmaceutical composition characterised in that substances of general formula I according to claim 1 or the acid addition salts thereof, and other pharmaceutically active substances and conventional galenic excipients and/or carriers are processed to form conventional pharmaceutical forms fo administration.

**Revendications pour les états contractants:**
BE, CH, DE, FR, GB, IT, LI, LU, NL, SE

1. Phénoxypropanolamines de formule générale (I)

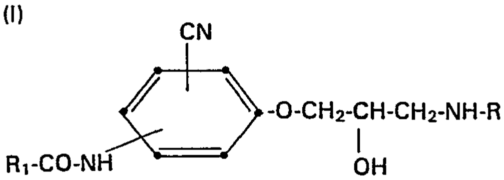

I

dans laquelle
$R_1$ représente un radical cycloalcoyle avec 3 à 6 atomes de C ou un radical phényle qui peut être éventuellement substitué par méthyle, méthoxy, chlore ou fluor, ou représente un radical phénoxyméthyle, qui peut être éventuellement substitué par méthyle, chlore ou brome, et
R représente un radical isopropyle ou sec.- ou tert.-butyle et leurs sels d'addition d'acides.

2. Le 1-[2-cyano-4-(3'-méthylphénoxyacétylamino)-phénoxy]-3-tert.-butylamino-2-propanol ou respectivement ses sels.

3. Le 1-(2-cyano-4-cyclobutanecarbonylamino--phénoxy)-3-tert.-butylamino-2-propanol ou respectivement ses sels.

4. Préparations pharmaceutiques contenant des substances de formule générale (I) selon la revendication 1 ou leurs sels d'addition d'acides, en combinaison avec des adjuvants et/ou excipients usuels.

5. Préparations pharmaceutiques contenant des substances de formule générale (I) selon la revendication 1 ou leurs sels d'addition d'acides, en combinaison avec d'autres substances actives pharmaceutiques ainsi que des adjuvants et/ou excipient usuels.

6. Composés selon la revendication 1 pour l'utilisation en tant que substances actives pour des médicaments pour le traitement et la prophylaxie des maladies des vaisseaux coronaires.

7. Procédé pour la préparation de préparations pharmaceutiques selon la revendication 4 ou 5, caractérisé en ce que l'on transforme des substances de formule générale (I) selon la revendication 1 ou leurs sels d'addition d'acides ainsi que des adjuvants et/ou excipients galéniques usuels en des formes d'utilisation pharmaceutiques usuelles.

8. Procédé pour la préparation de composés de formule générale (I) selon la revendication 1, caractérisé en ce que

a) on fait réagir un composé de formule générale (II)

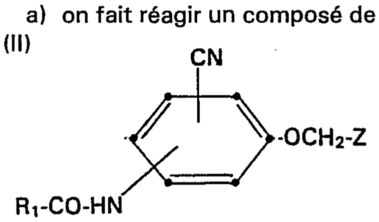

II

dans laquelle $R_1$ est défini comme dans la formule (I) et Z représente le groupe 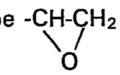 ou $-CHOH-CH_2-$ -Hal (Hal = halogène),
avec une amine de formule générale (III)

$$NH_2-R \qquad (III)$$

dans laquelle R est défini comme dans la formule (I), ou en ce que

b) on hydrolyse un dérivé d'oxazolidine de formule générale (IV)

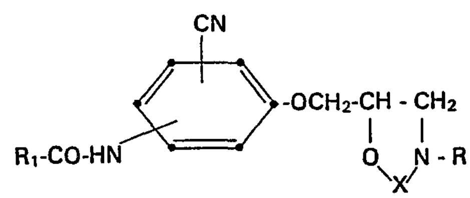

IV

dans laquelle $R_1$ et R sont définis comme dans la revendication 1 et X représente un groupe -CO-, $-CH_2-$ ou -CH-alcoyle inférieur, ou en ce que

c) on fait réagir un composé de formule générale (V)

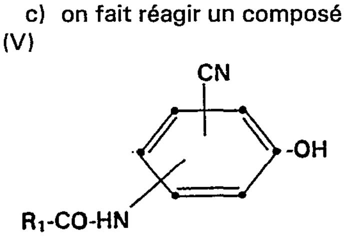

V

dans laquelle $R_1$ est défini comme dans la formule I (ou un sel de ce composé) avec un dérivé d'azétidinol de formule générale (VI)

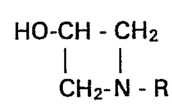

VI

dans laquelle R est défini comme dans la formule (I), et en ce que l'on transforme les composés obtenus selon l'un des procédés a) à c), si on le désire, en leurs sels d'addition d'acides.

**Revendications pour l'état contractant:** AT

1. Procédé pour la préparation de phénoxypropanolamines de formule générale (I)

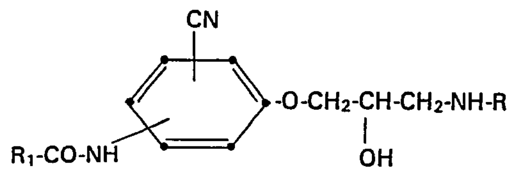

I

dans laquelle
$R_1$ représente un radical cycloalcoyle avec 3 à 6 atomes de C ou un radical phényle qui peut être éventuellement substitué par méthyle, méthoxy, chlore ou fluor, ou représente un radical phénoxyméthyle, qui peut être éventuellement substitué par méthyle, chlore ou brome, et
R représente un radical isopropyle ou sec.- ou tert.-butyle et leurs sels d'addition d'acides, caractérisé en ce que

a) on fait réagir un composé de formule générale (II)

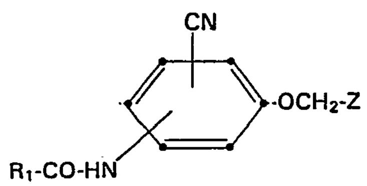

II

dans laquelle $R_1$ est défini comme dans la formule (I) et Z représente le groupe 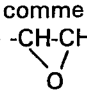 ou $-CHOH-CH_2-$ -Hal (Hal = halogène),
avec une amine de formule générale (III)

$NH_2$-R (III)

dans laquelle R est défini comme dans la formule (I), ou en ce que

b) on hydrolyse un dérivé d'oxazolidine de formule générale (IV)

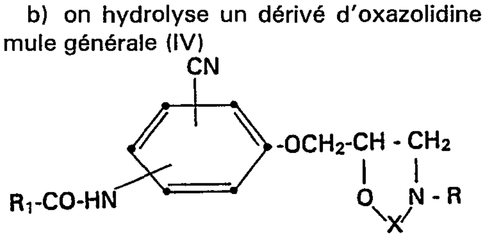

IV

dans laquelle $R_1$ et R sont définis comme dans la formule I et X représente un groupe -CO-, $-CH_2-$ ou -CH-alcoyle inférieur, ou en ce que

c) on fait réagir un composé de formule générale (V)

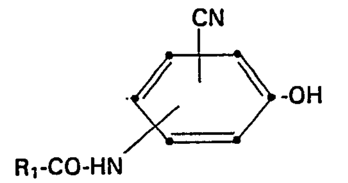

V

dans laquelle $R_1$ est défini comme dans la formule I (ou un sel de ce composé) avec un dérivé d'azétidinol de formule générale (VI)

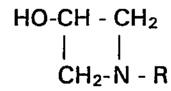

VI

dans laquelle R est défini comme dans la formule (I), et en ce que l'on transforme les composés obtenus selon l'un des procédés a) à c), si on le désire, en leurs sels d'addition d'acides.

2. Procédé selon la revendication 1, caractérisé en ce que l'on prépare le 1-[2-cyano-4-(3'-méthylphénoxyacétylamino)-phénoxy]-3-tert.-butylamino-2-propanol ou ses sels.

3. Procédé selon la revendication 1, caractérisé en ce que l'on prépare le 1-(2-cyano-4-cyclobutanecarbonylamino-phénoxy)-3-tert.-butylamino-2-propanol ou respectivement ses sels.

4. Procédé pour l'obtention de préparations pharmaceutiques, caractérisé en ce que l'on transforme des substances de formule générale (I) selon la revendication 1 ou respectivement leurs sels d'addition d'acides ainsi que des adjuvants et/ou excipients galéniques usuels en formes d'utilisations pharmaceutiques usuelles.

5. Procédé pour l'obtention de préparations pharmaceutiques, caractérisé en ce que l'on transforme des substances de formule générale (I) selon la revendication 1 ou respectivement leurs sels d'addition d'acides avec d'autres substances actives pharmaceutiques ainsi que des adjuvants et/ou excipients galéniques usuels en formes d'utilisations pharmaceutiques usuelles.